(19) **Europäisches Patentamt / European Patent Office / Office européen des brevets**

(11) **EP 4 061 231 B1**

(12) **EUROPEAN PATENT SPECIFICATION**

(45) Date of publication and mention of the grant of the patent:
**10.01.2024 Bulletin 2024/02**

(21) Application number: **20810928.0**

(22) Date of filing: **19.11.2020**

(51) International Patent Classification (IPC):
*A61B 8/00* (2006.01)   *G05B 13/02* (2006.01)
*G06N 3/02* (2006.01)   *A61B 8/08* (2006.01)
*A61B 5/06* (2006.01)

(52) Cooperative Patent Classification (CPC):
**A61B 8/4245; A61B 8/085; A61B 8/0866; A61B 8/0883; A61B 8/46; A61B 8/461; A61B 8/465; A61B 8/466; A61B 8/468; A61B 8/469; A61B 8/5207; A61B 8/5223; G06N 3/045; G06N 3/084; G06N 7/01;**   (Cont.)

(86) International application number:
**PCT/EP2020/082646**

(87) International publication number:
**WO 2021/099449 (27.05.2021 Gazette 2021/21)**

(54) **INTELLIGENT MEASUREMENT ASSISTANCE FOR ULTRASOUND IMAGING AND ASSOCIATED DEVICES, SYSTEMS, AND METHODS**

INTELLIGENTE MESSUNTERSTÜTZUNG FÜR ULTRASCHALLBILDGEBUNG UND ZUGEHÖRIGE VORRICHTUNGEN, SYSTEME UND VERFAHREN

AIDE À LA MESURE INTELLIGENTE POUR IMAGERIE PAR ULTRASONS ET DISPOSITIFS, SYSTÈMES ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.11.2019 US 201962939164 P**

(43) Date of publication of application:
**28.09.2022 Bulletin 2022/39**

(73) Proprietor: **Koninklijke Philips N.V.**
**5656 AG Eindhoven (NL)**

(72) Inventors:
• **MERAL, Faik, Can**
**5656 AE Eindhoven (NL)**
• **BHARAT, Shyam**
**5656 AE Eindhoven (NL)**
• **TOPOREK, Grzegorz, Andrzej**
**5656 AE Eindhoven (NL)**
• **BALICKI, Marcin, Arkadiusz**
**5656 AE Eindhoven (NL)**

• **SRINIVASA NAIDU, Raghavendra**
**5656 AE Eindhoven (NL)**

(74) Representative: **Philips Intellectual Property & Standards**
**High Tech Campus 52**
**5656 AG Eindhoven (NL)**

(56) References cited:
**US-A1- 2008 221 446    US-A1- 2017 325 783**

• **HANSEGARD J ET AL: "Constrained Active Appearance Models for Segmentation of Triplane Echocardiograms", IEEE TRANSACTIONS ON MEDICAL IMAGING, IEEE SERVICE CENTER, PISCATAWAY, NJ, US, vol. 26, no. 10, 1 October 2007 (2007-10-01), pages 1391-1400, XP011193206, ISSN: 0278-0062, DOI: 10.1109/TMI.2007.900692**

**(Cont. next page)**

(52) Cooperative Patent Classification (CPC): (Cont.)
G05B 2219/37269

**Description**

**TECHNICAL FIELD**

The present disclosure relates generally to ultrasound imaging and, in particular, to providing automated measurements of anatomical features from ultrasound images.

**BACKGROUND**

Ultrasound imaging systems are widely used for medical imaging. For example, a medical ultrasound system may include an ultrasound transducer probe coupled to a processing system and one or more display devices. The ultrasound transducer probe may include an array of ultrasound transducer elements that transmit acoustic waves into a patient's body and record acoustic waves reflected from the internal anatomical structures within the patient's body, which may include tissues, blood vessels, and internal organs. The transmission of the acoustic waves and/or the reception of reflected acoustic waves or echo responses can be performed by the same set of ultrasound transducer elements or different sets of ultrasound transducer elements. The processing system can apply beamforming, signal processing, and/or imaging processing to the received echo responses to create an image of the patient's internal anatomical structures. The image may be presented to a clinician in the form of a brightness-mode (B-mode) image, where each pixel of the image is represented by a brightness level or intensity level corresponding to the echo strength.

Ultrasound imaging can be used for diagnostic examination, interventions, and/or treatment. Additionally, ultrasound imaging can be used as a quantification tool for measurements of anatomical features. For example, during an obstetric examination, ultrasound imaging can be used to capture images of a fetus carried by a pregnant woman and a clinician may evaluate fetal growth based on measurements of the fetal head or other parts of the fetus from the ultrasound images. Alternatively, during a cardiac examination, ultrasound imaging can be used to capture images of a patient's heart and a clinician may perform quantitative cardiac measurements from the ultrasound images.

To perform measurements from ultrasound images, a clinician may place measurement points (e.g., calipers) on the area of interest within an image. The processing system may be equipped with software that can determine measurements for the area of interest based on the measurement points. The measurement points may operate as end points where measurement is to be made. Various ultrasound measurement tools are available for today's ultrasound transducers, such as a depth of a point in the image with respect to the transducer surface, a distance between two points in the region of interest (ROI), a diameter of a circle based on the points placed on a perimeter of the ROI, long-axis and/or short-axis measurements of an ellipsoid based on the points placed on the perimeter of the ROI. However, all of these measurements are one-dimensional or two-dimensional (1D/2D) representations of three-dimensional (3D) anatomical structure measured from 2D images. As such, the measurement itself and the image it was derived from are from a lower dimensional representation of the actual anatomical structure. Sonographers are aware of this limitation and may therefore use additional features in the image to ensure that the imaging plane is a representative cross-section of the 3D anatomy to be measured. Thus, measurements can vary depending on the sonographers and the process of locating the optimal measurement plane can be time-consuming.

US 2017/325783 A1 discloses an approach for determining dimensions of structures in medical images; and may be considered to disclose an ultrasound imaging system comprising: a processor circuit in communication with an ultrasound transducer array volume of a patient's anatomy including an anatomical feature; obtain first measurement data of the anatomical feature in a first image of the set of images; generate second measurement data for the anatomical feature in one or more images of the set of images by propagating the first measurement data from the first image to the one or more images; and output, to a display in communication with the processor circuit, the second measurement data for the anatomical feature.

US 2008/221446 A1 discloses an approach for tracking points in an ultrasound image.

**SUMMARY**

The invention is defined by the claims. There remains a clinical need for improved systems and techniques for providing efficient and accurate ultrasound image-based anatomical feature measurements. Embodiments of the present disclosure provide techniques for automated anatomical feature measurements from ultrasound images. In the disclosed embodiments, an ultrasound imaging system may utilize a tracked ultrasound probe to acquire a set of image frames around an anatomical feature of interest (e.g., a fetal head or a cardiac chamber). The ultrasound probe may be fitted with an inertial measurement tracker (e.g., including an accelerometer, gyroscope, and/or sensors) that can provide positional and/or motion information of the ultrasound probe during image acquisition. Additionally, the ultrasound probe may include markers that can be tracked by an external electromagnetic (EM) tracking and/or optical tracking system. A three-dimensional (3D) volume enclosing the anatomical feature of interest may be reconstructed based on the acquired images and the tracked positional and/or motion information of the probe. The 3D volume may enclose an optimal measurement plane for measuring the anatomical feature of interest. A clinician may place measurement markers (e.g., calipers) on a first image of the images for a target measurement (e.g., a maximum length or diameter of a fetal head or a cardiac chamber width). The system may provide the clinician with measurement assistance by utilizing a prediction network (e.g., a deep learning network) to propagate the measurement markers from the first image to other acquired images based on the reconstructed 3D volume. Additionally, the prediction network may be trained to create multi-planar reconstructions (MPRs) from the reconstructed 3D volume and propagate the measurement markers from the first image to all the MPRs. Thus, the prediction network may provide a cross-plane (e.g., an MPR) for obtaining an optimal measurement for the feature of interest. Further, the prediction network may be trained to segment the anatomical feature of interest from the images and perform measurements based on the segmented feature. The prediction network may output a final measurement based on measurements obtained from all the images, statistics of the measurements, and/or a confidence of the measurements.

In one embodiment, an ultrasound imaging system including a processor circuit in communication with an ultrasound transducer array, the processor circuit configured to receive, from the ultrasound transducer array, a set of images of a three-dimensional (3D) volume of a patient's anatomy including an anatomical feature; obtain first measurement data of the anatomical feature in a first image of the set of images; generate second measurement data for the anatomical feature in one or more images of the set of images by propagating the first measurement data from the first image to the one or more images; and output, to a display in communication with the processor circuit, the second measurement data for the anatomical feature.

In some aspects, the system may also include where the processor circuit configured to obtain the first measurement data is configured to receive, from a user interface in communication with the processor circuit, the first measurement data including at least two measurement markers across the anatomical feature on the first image. The set of images is associated with a plurality of imaging planes across the 3D volume of the patient's anatomy including the anatomical feature. The processor circuit is configured to propagate the first measurement data from the first image to the one or more images based on positional data of the ultrasound transducer array with respect to the plurality of imaging planes. In some aspects, the system may also include where the processor circuit configured to generate the second measurement data is configured to determine 3D spatial data for the first image and the one or more images based on the positional data of the ultrasound transducer array; and propagate the first measurement data from the first image to the one or more images based on the 3D spatial data. In some aspects, the system may also include a probe including the ultrasound transducer array and an inertial measurement tracker, where the processor circuit is configured to receive, from the inertial measurement tracker, inertial measurement data associated with the ultrasound transducer array and the plurality of imaging planes, and where the processor circuit configured to determine the 3D spatial data is configured to determine the positional data of the ultrasound transducer array with respect to the plurality of imaging planes based on the inertial measurement data and an inertial-measurement-to-image transformation. In some aspects, the system may also include where the processor circuit is configured to generate third measurement data for the anatomical feature based on the first measurement data and the second measurement data, where the third measurement data is associated with at least one of a first imaging plane of the plurality of imaging planes or a second imaging plane within the 3D volume different from the plurality of imaging planes; and output, to the display, the third measurement data. In some aspects, the system may also include where the second imaging plane intersects the first imaging plane. In some aspects, the system may also include where the third measurement data includes at least one of the second measurement data, a distance between two measurement markers across the anatomical feature, a confidence metric of the first measurement data, a confidence metric of the second measurement data, a mean value of the first measurement data and the second measurement data, a variance of the first measurement data and the second measurement data, or a standard deviation of the first measurement data and the second measurement data. In some aspects, the system may also include a user interface in communication with the processor circuit and configured to provide a selection associated with the third measurement data. In some aspects, the system may also include where the processor circuit configured to generate the second measurement data for the anatomical feature in the one or more images is configured to propagate the first measurement data from the first image to the one or more images based on image segmentation. In some aspects, the system may also include where the processor circuit configured to generate the second measurement data for the anatomical feature in the one or more images is configured to propagate the first measurement data from the first image to the one or more images using a predictive network trained for at least one of an image segmentation or a feature measurement. In some aspects, the system may also include where the predictive network is trained on a set of image-measurement pairs for the feature measurement, and where each image-measurement pair of the set of image-measurement pair includes an image in a sequence of images of a 3D anatomical volume and a measurement of a feature of the 3D anatomical volume for the image. In some aspects, the system may also include where the predictive network is trained on a set of image-segment pairs for the image segmentation, where each image-segment pair of the set of image-segment pair includes an image in a sequence of images of a 3D anatomical volume and a segment of a feature of the 3D anatomical volume for the image. In some aspects, the system may also include where the anatomical feature includes a fetal head, and where the first measurement data and the second measurement data are associated with at least one of a circumference of the fetal head or a length of the fetal head. In some aspects, the system may also include where anatomical feature includes a left ventricle, and where the first measurement data and the second measurement data are associated with at least one of a width, a height, an area, or a volume of the left ventricle.

In one embodiment, a method of ultrasound imaging, including receiving, at a processor circuit in communication with an ultrasound transducer array, a set of images of a three-dimensional (3D) volume of a patient's anatomy including an anatomical feature; obtaining first measurement data of the anatomical feature in a first image of the set of images; generating, at the processor circuit, second measurement data for the anatomical feature in one or more images of the set of images by propagating the first measurement data from the first image to the one or more images; and outputting, to a display in communication with the processor circuit, the second measurement data for the anatomical feature.

In some aspects, the method may also include where the obtaining the first measurement data includes receiving, from a user interface in communication with the processor circuit, the first measurement data including at least two measurement markers across the anatomical feature. The set of images is associated with a plurality of imaging planes across the 3D volume of the patient's anatomy including the anatomical feature. The propagating the first measurement data from the first image to the one or more images is based on positional data of the ultrasound transducer array with respect to the plurality of imaging planes. Optionally, the generating the second measurement data includes determining 3D spatial data for the first image and the one or more images based on positional data of the ultrasound transducer array with respect to the plurality of imaging planes; and propagating the first measurement data from the first image to the one or more images based on the 3D spatial data. In some aspects, the method may also include receiving, from an inertial measurement tracker in communication with the processor circuit, inertial measurement data associated with the ultrasound transducer array, and determining positional data of the ultrasound transducer array with respect to the first image and the one or more images based on the inertial measurement data and an inertial-measurement-to-image transformation.

Additional aspects, features, and advantages of the present disclosure will become apparent from the following detailed description.

## BRIEF DESCRIPTION OF THE DRAWINGS

Illustrative embodiments of the present disclosure will be described with reference to the accompanying drawings, of which:

FIG. 1 is a schematic diagram of an ultrasound imaging system, according to aspects of the present disclosure.
FIG. 2 is a schematic diagram of an automated ultrasound image-based measurement scheme, according to aspects of the present disclosure.
FIG. 3 is a schematic diagram of an automated ultrasound image-based measurement scheme, according to aspects of the present disclosure.
FIG. 4 is a schematic diagram of an automated ultrasound image-based measurement scheme, according to aspects of the present disclosure.
FIG. 5 is a schematic diagram of an automated deep learning, ultrasound image-based measurement scheme, according to aspects of the present disclosure.
FIG. 6 is a schematic diagram of a deep learning network configuration for ultrasound image-based measurement, according to aspects of the present disclosure.
FIG. 7 is a schematic diagram of a deep learning network training scheme ultrasound image-based measurement, according to aspects of the present disclosure.
FIG. 8 is a schematic diagram of an automated deep-learning, ultrasound image-based measurement scheme, according to aspects of the present disclosure.
FIG. 9 is a schematic diagram of a user interface for an automated ultrasound image-based measurement system, according to aspects of the present disclosure.
FIG. 10 is a schematic diagram of a processor circuit, according to embodiments of the present disclosure.
FIG. 11 is a flow diagram of a deep learning, ultrasound image-based measurement method, according to aspects of the present disclosure.

## DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

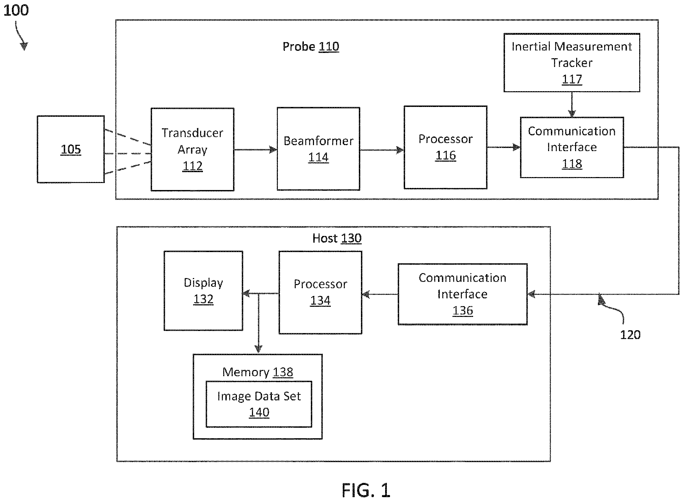

FIG. 1 is a schematic diagram of an ultrasound imaging system 100, according to aspects of the present disclosure. The system 100 is used for scanning an area or volume of a patient's body. The system 100 includes an ultrasound imaging probe 110 in communication with a host 130 over a communication interface or link 120. The probe 110 includes a transducer array 112, a beamformer 114, a processor circuit 116, and a communication interface 118. The host 130 includes a display 132, a processor circuit 134, and a communication interface 136.

In an exemplary embodiment, the probe 110 is an external ultrasound imaging device including a housing configured for handheld operation by a user. The transducer array 112 can be configured to obtain ultrasound data while the user grasps the housing of the probe 110 such that the transducer array 112 is positioned adjacent to and/or in contact with a patient's skin. The probe 110 is configured to obtain ultrasound data of anatomy within the patient's body while the probe 110 is positioned outside of the patient's body. In some embodiment, the probe 110 can be an external ultrasound probe suitable for fetal examination. In some other embodiments, the probe 110 can be a transthoracic (TTE) probe or a trans-esophageal (TEE) ultrasound probe suitable for cardiac examination.

The transducer array 112 emits ultrasound signals towards an anatomical object 105 of a patient and receives echo signals reflected from the object 105 back to the transducer array 112. The ultrasound transducer array 112 can include any suitable number of acoustic elements, including one or more acoustic elements and/or plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of acoustic elements (e.g., one or more rows, one or more columns, and/or one or more orientations) that can be uniformly or independently controlled and activated. The transducer array 112 can be configured to obtain one-dimensional, two-dimensional, and/or three-dimensional images of patient anatomy. In some embodiments, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The object 105 may include any anatomy, such as blood vessels, nerve fibers, airways, mitral leaflets, cardiac structure, abdominal tissue structure, kidney, and/or liver of a patient and/or a fetus within a pregnant mother that is suitable for ultrasound imaging examination. In some embodiments, the object 105 may include at least a portion of a patient's heart, lungs, and/or skin. The present disclosure can be implemented in the context of any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood vessels, blood, chambers or other parts of the heart, a pregnant mother's womb, and/or other systems of the body. In some embodiments, the object 105 may include malignancies such as tumors, cysts, lesions, hemorrhages, or blood pools within any part of human anatomy. The anatomy may be a blood vessel, as an artery or a vein of a patient's vascular system, including cardiac vasculature, peripheral vasculature, neural vasculature, renal vasculature, and/or any other suitable lumen inside the body. In addition to natural structures, the present disclosure can be implemented in the context of man-made structures such as, but without limitation, heart valves, stents, shunts, filters, implants and other devices.

The beamformer 114 is coupled to the transducer array 112. The beamformer 114 controls the transducer array 112, for example, for transmission of the ultrasound signals and reception of the ultrasound echo signals. The beamformer 114 provides image signals to the processor circuit 116 based on the response of the received ultrasound echo signals. The beamformer 114 may include multiple stages of beamforming. The beamforming can reduce the number of signal lines for coupling to the processor circuit 116. In some embodiments, the transducer array 112 in combination with the beamformer 114 may be referred to as an ultrasound imaging component.

The processor circuit 116 is coupled to the beamformer 114. The processor circuit 116 may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor circuit 134 may also be implemented as a combination of computing devices, e.g., a combination of a DSP and a microprocessor, a plurality of microprocessors, one or more microprocessors in conjunction with a DSP core, or any other such configuration. The processor circuit 116 is configured to process the beamformed image signals. For example, the processor circuit 116 may perform filtering and/or quadrature demodulation to condition the image signals. The processor circuit 116 and/or 134 can be configured to control the array 112 to obtain ultrasound data associated with the object 105.

The communication interface 118 is coupled to the processor circuit 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 nay be a wireless link, such as an ultra-wideband (UWB) link, an Institute of Electrical and Electronics Engineers (IEEE) 802.11 WiFi link, or a Bluetooth link.

At the host 130, the communication interface 136 may receive the image signals. The communication interface 136 may be substantially similar to the communication interface 118. The host 130 may be any suitable computing and display device, such as a workstation, a personal computer (PC), a laptop, a tablet, or a mobile phone.

The processor circuit 134 is coupled to the communication interface 136. The processor circuit 134 may be implemented as a combination of software components and hardware components. The processor circuit 134 may include a central processing unit (CPU), a graphics processing unit (GPU), a digital signal processor (DSP), an application-specific integrated circuit (ASIC), a controller, a FPGA device, another hardware device, a firmware device, or any combination thereof configured to perform the operations described herein. The processor circuit 134 may also be implemented as a combination of computing devices, e.g., a combination of a DSP and a microprocessor, a plurality of microprocessors, one or more microprocessors in conjunction with a DSP core, or any other such configuration. The processor circuit 134 can be configured to generate image data from the image signals received from the probe 110. The processor circuit 134 can apply advanced signal processing and/or image processing techniques to the image signals. In some embodiments, the processor circuit 134 can form three-dimensional (3D) volume image from the image data. In some embodiments, the processor circuit 134 can perform real-time processing on the image data to provide a streaming video of ultrasound images of the object 105.

The display 132 is coupled to the processor circuit 134. The display 132 may be a monitor or any suitable display. The display 132 is configured to display the ultrasound images, image videos, and/or any imaging information of the object 105 and/or the medical device 108.

The system 100 may be used to assist a sonographer in performing measurements from acquired ultrasound images. In some aspects, the system 100 can capture a sequence of ultrasound images of the object 105. The clinician may be interested in determining a measurement of a certain anatomical feature of the object 105. In an example, a sonographer may perform a 2D transthoracic cardiac ultrasound scan of the object 105 including a patient's cardiac structure and perform quantitative structural and/or functional measurements of the cardiac structure. For instance, the sonographer may perform linear measurements of a during systole and diastole and/or LV volume measurements using techniques, such as biplane method of discs, to estimate functional parameters (e.g., Ejection Fraction (EF)) during the scan. Similarly, the sonographer may perform right ventricle (RV) structural measurements to assess the RV function during the scan. For instance, right ventricular outflow tract (RVOT) is measured in proximal and distal directions. Additionally, structural size measurements such as ventricular equality referring to the relative size of the left atrium to the right atrium, aortic root diameter, inferior vena cava diameter, can be made during echocardiography scans. To obtain accurate measurements, it is important to avoid foreshortening. Foreshortening refers to a situation where the 2D ultrasound plane does not cut through the apex of the cardiac structure. Foreshortening can yield erroneous measurements. In another example, a sonographer may perform fetal imaging to obtain measurements of a fetal head circumference, which is a key measurement indicative of the fetal growth. Thus, accurate and precise measurements of the fetal head circumference is important. To obtain accurate and precise measurements of a fetal head circumference, the measurement is to be performed in axial cross-sectional plane, which is the plane that goes through the baby's head perpendicular to its feet-head axis. Additionally, the measurement is to be performed at a level that maximizes the measurement. A fetal head circumference measurement made on an arbitrary imaging plane can be misleading. To ensure a correct or optimal measurement imaging plane is captured, the sonographer may seek the presence of cranial features in the images that indicate the correct imaging plane. Although the sonographer may seek additional anatomical features to ensure that measurements are made at a correct measurement plane, the scan time can be long and the resulted measurements can be user-dependent.

According to embodiments of the present disclosure, the system 100 is further configured to provide automated ultrasound image-based measurements by using a tracked ultrasound probe to acquire ultrasound images (e.g., 2D ultrasound images) at and/or around an optimal measurement plane and using the tracking information to create a 3D volume enclosing the measurement plane. In some aspects, the probe 110 may include an inertial measurement tracker 117. The inertial measurement tracker 117 may include accelerometers, gyroscopes, and/or sensors to acquire and track motion of the probe 110 while the object 105 is scanned. The system 100 may additionally include an external tracking system, which may be based on electromagnetic (EM) tracking and/or optical tracking, and the probe 110 may include markers that can be tracked by the external tracking system to provide positional and/or information of the probe 110. The processor circuit 134 may create a 3D volume of the object 105 or a 3D spatial data set that defines the object 105 in a 3D space based on the tracking information and the acquired images. The 3D spatial information can allow for more accurate measurements with the aid of an artificial intelligence (AI) or deep leaning-based agents.

In some aspects, the processor circuit 134 may implement one or more deep learning-based prediction networks trained to identify a region of interest on an ultrasound image for measurements, propagate user identified measurement locations from one image to neighboring images, create multi-planar reconstructions (MPRs) for cross-plane measurements, and/or segment the anatomy of interest from images for making automated measurements. Mechanisms for providing automated measurements from ultrasound images are described in greater detail herein.

In some aspects, the system 100 can be used for collecting ultrasound images to form training data set for deep learning network training. For example, the host 130 may include a memory 138, which may be any suitable storage device, such as a cache memory (e.g., a cache memory of the processor circuit 134), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory. The memory 138 can be configured to store an image data set 140 to train the series of prediction or deep learning networks for providing automated ultrasound imaged-based measurements. Mechanisms for training the prediction or deep learning networks are described in greater detail herein.

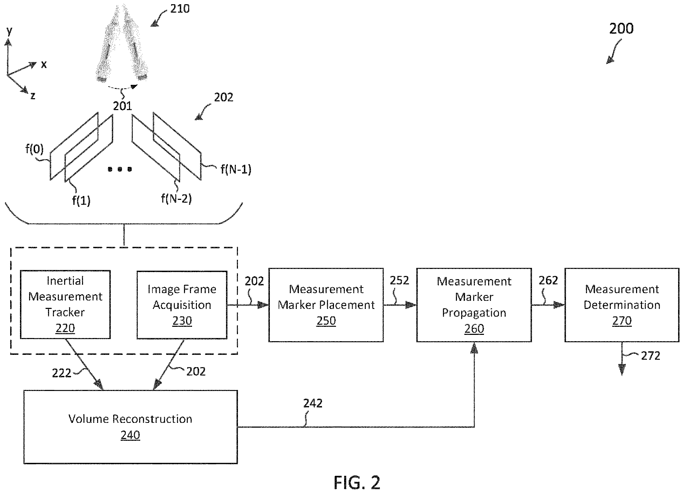

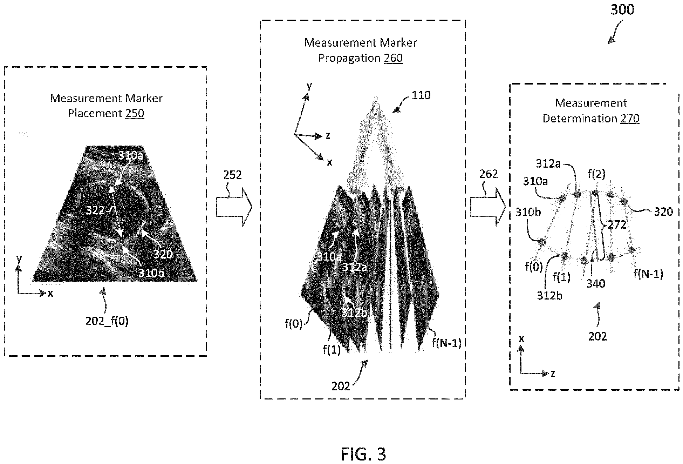

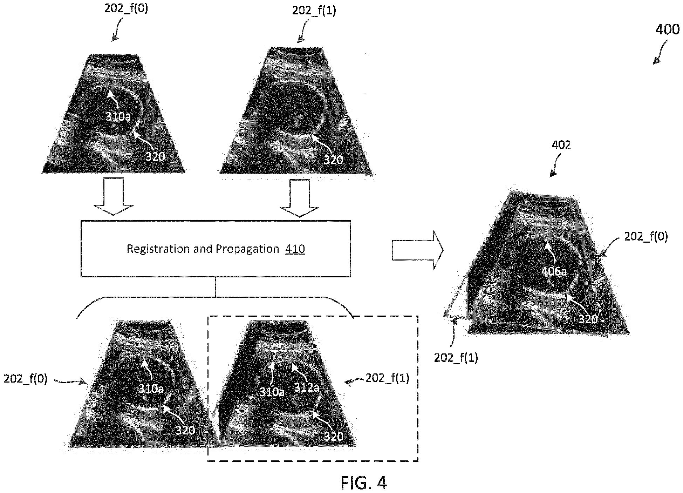

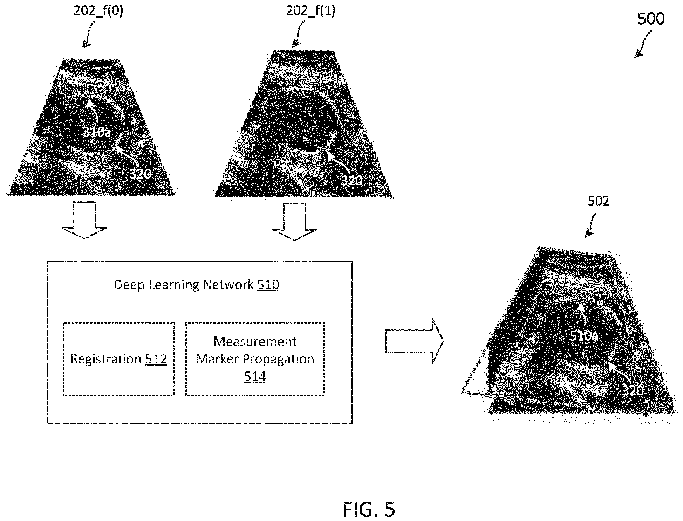

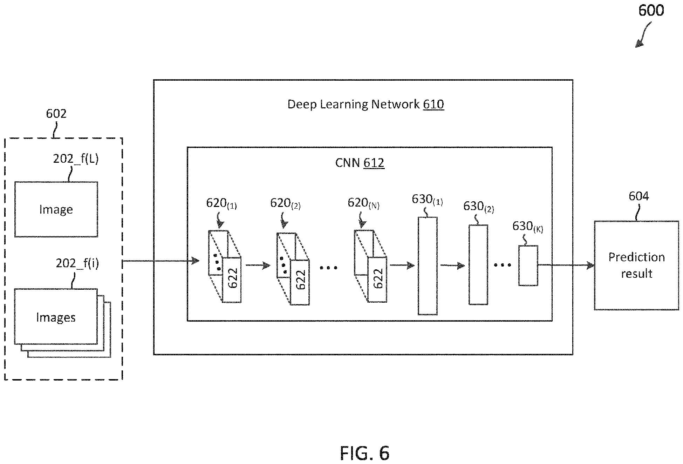

FIGS. 2-6 collectively illustrate mechanisms for automated measurements from ultrasound images. FIG. 2 is a schematic diagram of an automated ultrasound image-based measurement scheme 200, according to aspects of the present disclosure. FIG. 3 is a schematic diagram of an automated ultrasound image-based measurement scheme 300, according to aspects of the present disclosure. FIG. 4 is a schematic diagram of an automated ultrasound image-based measurement scheme 400, according to aspects of the present disclosure. FIG. 5 is a schematic diagram of an automated deep learning, ultrasound image-based measurement scheme 500, according to aspects of the present disclosure. FIG. 6 is a schematic diagram of a deep learning network configuration 600 for ultrasound image-based measurement, according to aspects of the present disclosure. The schemes 200, 300, 400, and 500 can be implemented by the system 100

Referring to FIG. 2, the scheme 200 includes an inertial measurement tracker 220, an image frame acquisition component 230, a volume reconstruction component 240, a measurement marker placement component 250, a measurement marker propagation component 260, and a measurement determination component 270. The inertial measurement tracker 220, the image frame acquisition component 230, the volume reconstruction component 240, the measurement marker placement component 250, the measurement marker propagation component 260, and the measurement determination component 270 may be implemented by a combination of hardware (e.g., including processing circuitry, logic, and/or gates) and/or software. In some instances, the volume reconstruction component 240, the measurement marker placement component 250, a measurement marker propagation component 260, and/or the measurement determination component 270 can be implemented by the processor circuit 134.

At a high level, the scheme 200 uses a tracked ultrasound probe 210 similar to the probe 110 to acquire a set of images 202 of a patient's anatomy (e.g., the object 105) around a measurement plane where a measurement for a feature of interest within the patient's anatomy can be made. In this regard, a sonographer or user may sweep the probe 210 around an area of interest where a measurement is to be made. The image frame acquisition component 230 acquires the images 202 as the probe 210 is swept as shown by the dashed arrow 201. The images 202 are shown as f(0), f(1), ..., f(N-2), and f(N-1). In some instances, the probe 210 may be a 1D ultrasound probe configured to acquire the 2D ultrasound images 202 and the sweeping may include physically sweeping the probe 210 around the area of interest in a 3D volume of the patient's anatomy. In some other instances, the probe 210 may be a 2D ultrasound probe capable of performing 3D imaging and the sweeping may include electronically steering ultrasound beams to acquire the 2D images 202 at various 2D imaging planes within the 3D volume. The image frame acquisition component 230 may be configured to acquire the images 202 at a certain frame rate. The image frame acquisition component 230 may provide the set of images 202 to the volume reconstruction component 240.

The inertial measurement tracker 220 is similar to the inertial measurement tracker 117, for example, including an accelerator, a gyroscope, and/or sensors, and may be located within the probe 210. The inertial measurement tracker 220 is configured to track motions of the probe 210 while the set of images 202 is acquired. The inertial measurement tracker 220 is configured to record positional information 222 of the probe 210 while the images 202 are acquired such that the locations or coordinates of the imaging plane for each image 202 are known for subsequent processing. For instance, at a time instant T1, the probe 210 may acquire the image 202 f(0) at a first imaging plane. At a next time instant T2, the probe 210 may acquire the image 202 f(1) at a second imaging plane. The positional information 222 may include translations and/or rotations that are applied to the probe 210 or beam steering between the time instant T1 and the time instant T2 such that the probe 210 may arrive at the second imaging plane. The positional information 222 is provided to the volume reconstruction component 240 for volume reconstruction.

In some aspects, the inertial measurement tracker 220 can provide six degrees of freedom (6DOF) in space with three axes of acceleration and three axes of rotational speed. While three-axis acceleration and three-axis gyroscope information may be sufficient for determining motions of the ultrasound probe 210 with respect to a reference coordinate system, additional information provided by other inertial measurement tracking system such as electromagnetic (EM) field readings, and/or optical field readings can improve the overall measurement accuracy. In this regard, the ultrasound probe 210 can be fitted with markers that can be tracked via EM-based tracking and/or optical tracking.

The inertial measurement tracker 220 may provide acceleration, translational, and/or rotational measurements in a local coordinate frame of the inertial measurement tracker 220 (with respect to axes of the inertial measurement tracker 220). However, these measurements can be noisy and may have a certain measurement bias. Various techniques can be applied to calculate pose information of the probe 210 in a global coordinate frame (e.g., a certain frame of reference) to provide more accurate positional information. In this regard, the probe 210 may be attached with sensors and the sensor information can be integrated with the acceleration, translational, and/or rotational measurement data to provide more accurate positional information. For instance, readings from the sensors can be used to calibrate the local coordinate system of the inertial measurement tracker 220 with respect to an image coordinate system in a 3D space as described in greater detail herein below. In some other instances, the scheme 200 can apply certain filtering operations and/or fusion algorithms to the positional information to reduce noise and/or bias to improve accuracy in the measurements. In some instances, in addition to the inertial measurement tracker 220, image-based tracking may be used to estimate the probe 210's position and/or motion (e.g., translations and/or rotations). For example, the image-based tracking may include deep learning-based algorithms that regress 6DOF poses based on extracted anatomical features. Alternatively, the image-based tracking may include traditional image processing algorithms, such as registration-based algorithms and/or speckle-tracking algorithms. In general, the scheme 200 may employ the positional information 222 obtained from the inertial measurement tracker 220 in conjunction with positional information measured by any other suitable tracking systems to determine the position of the probe 210 with respect to the global coordinate frame.

During the acquisition, the system may continuously buffer the images 202 in a memory (e.g., the memory 138) until the user freezes or stops the acquisition. The volume reconstruction component 240 is configured to determine 3D pose information for each image 202 in a 3D space. In other words, the volume reconstruction component 240 may determine a relative position between two acquired images 202. In this regard, the volume reconstruction component 240 may determine the 3D image pose by multiplying the pose of the sensor or the inertial measurement tracker 220 with a transformation matrix as shown below:

$$I(x, y, z, t) = M_T \times S(x, y, z, t), \qquad (1)$$

where $I(x, y, z, t)$ represents the 3D pose of an image 202, $S(x, y, z, t)$ represents the 3D pose of the sensor or the inertial measurement tracker 220, $M_T$ represents the transformation matrix, $(x, y, z)$ are unit vectors of the rotational component of the transformation in a 3D space with respect to a certain frame of reference, and $t$ represents the translation. The volume reconstruction component 240 may compute the pose of the sensor or the inertial measurement tracker 220 based on the positional information 222, for example, using fusion algorithms. The transformation matrix, $M_T$, can be obtained through a calibration procedure where sensor location/orientation and image orientation are measured with respect to each other through actual measurements and/or computer-aided design (CAD) images.

In some aspects, the calibration can be performed for each probe 210 during a set up phase prior to imaging or during a manufacturing stage. The transformation matrix, $M_T$, transforms a coordinate system of the transducer (e.g., the transducer array 112) on the probe 210 to a coordinate system (defined by the inertial measurement tracker 220) of the image 202. For instance, image orientation can be measured from the transducer orientation. The calibration may determine translations and/or rotations for the transformation matrix, $M_T$, so that the application of the transformation matrix, $M_T$, may convert an inertial measurement tracker 220 pose to an image pose. After obtaining the 3D image pose for each image 202, coordinates of all points (e.g., pixels) in an image 202 with respect to other images 202 are known and a 3D placement of all the points in the set of images 202 are known.

As an example, each image 202 may include 256 pixels, each represented by an intensity value at a point defined by an (x, y) homogenous coordinate, which may be transformed to the transducer space using a first transformation matrix. The first transformation matrix is a 3x3 matrix including 2x2 rotations (at entries (1,1), (1,2), (2,1), (2,2) of the matrix) and 2 translations (at the last column of the matrix). The 2x2 rotations correspond to the in-plane rotation component of the image (i.e., around the z-axis, perpendicular to the image). Any vector on the image 202 can be described using the first transformation matrix and a reference point, which may be at the end of the probe 210 where the image starts. The image reference point and the sensor coordinate system is also related to each other by a known (or measurable) second transformation matrix (e.g., $M_T$). Since the image reference point and the sensor coordinate system are in a 3-dimensional space, the second transformation matrix is a 4x4 matrix, with 3x3 rotations and 3 translations. When the probe 210 is moved from one imaging plane (e.g., the imaging plane of f(0) at time instant T1) to a next imaging plane (e.g., the imaging plane of f(1) at time instant T2), the motion is represented by a third transformation matrix (e.g., a 4x4 matrix) from the sensor readings (e.g., the positional information 222) corresponding to how much the sensor (or the inertial measurement tracker 220) moved and in which direction the sensor moved. The third transformation matrix can be multiplied with the second transformation matrix to obtain an amount of movement moved by the image reference point (the end of the probe) during this motion. Subsequently, the product of the second and third transformation matrices can be multiplied with the first transformation matrix to obtain the motion of a certain pixel location experiences. Similarly, a point p1 in a first image 202 (e.g., f(0)) and a point p2 in a second image 202 (e.g., f(1)) can be related to each other using the same set of transformation matrices. Thus, the volume reconstruction component 240 produces a 3D volume or 3D spatial data set 242 including an x-y-z coordinate in a 3D space and a corresponding intensity value for each pixel in each image 202.

In some other instances, the volume reconstruction component 240 may construct the 3D volume (e.g., the 3D spatial data set 242) using a deep learning network (e.g., a CNN) that regresses the pose of the image with respect to a local coordinate system of a specific anatomical structure on which the network is trained on. In yet some other instances, the relative distance between two images 202 (e.g., the z-coordinate value) can be determined based on speckle statistics and speckle decorrelation, using an image acquisition frame rate and a beam focusing/steering configuration without the tracking measurements and/or transformation. This method may require some initial calibrations for image based tracking by the transducer manufacturer such as speckle calibration curves.

The image frame acquisition component 230 may further provide the image 202 to the measurement marker placement component 250. The measurement marker placement component 250 may place measurement markers or calipers (shown as 310a and 310b in FIG. 3) on an image 202 (e.g., the image f(0)), where measurements may be made. The image 202 f(0) are shown as 202_f(0). The measurement marker placement component 250 may output first measurement data 252 including the image 202_f(0) and information associated with the measurement markers. In some instances, the placement of the markers can be received via a user input. In other words, a sonographer performing the scan may determine the locations where the markers may be placed for measurements. In some other instances, the placement of the markers can be generated by a deep learning network trained for measurement point identification.

As an example, the images 202 are acquired during a fetal examination, where the images 202 may include a view of a fetal head within a mother's womb. The measurement markers can be placed on the image for fetal head measurements as discussed below in FIG. 3.

Referring to FIG. 3, the measurement marker placement component 250 may operate on the image 202, f(0). The image 202 includes the view of a fetal head 320 and markers 310a and 310b placed on the circumference of the fetal head 320 such that a distance 322 between the markers 310a and 310b may represent a diameter of the fetal head 320. The measurement markers 310a and 310b may be in the form of measurement points as shown. In some other instances, the measurement markers 310a and 310b may be in the form of lines, crosses, and/or any other suitable forms, symbols, and/or shapes. As discussed above, the placement of the markers 310a and 310b can be performed by a sonographer, and thus the measurement marker placement component may simply receive the locations of the markers 310a and 310b from the sonographer.

Returning to FIG. 2, after placing measurement markers 310a and 310b on the image 202_f(0), the measurement marker propagation component 260 is configured to propagate the measurement markers 310a and 310b from the image 202_f(0), to other neighboring images 202 stored in the buffer memory.

Referring to FIG. 3, the measurement marker propagation component 260 propagates the measurement markers 310a and 310b selected for the image 202_f(0) to the other images 202 (e.g., f(1), f(2), ... , f(N-1)) in the set. In this regard, the measurement marker propagation component 260 may first register a neighboring image 202 (e.g., the image f(1)) with the image 202_f(0) where the markers are placed, followed by transferring the measurement markers 310a and 310b to the neighboring image 202_f(1). Image registration may refer to the spatial alignment of one image to another image as discussed in greater detail below in FIGS. 4 and 5. The propagation of the measurement markers 310a and 310b may continue, where the propagated measurement for the image 202_(f1) may serve as the original measurement and propagated onto the next image 202 (e.g., the image f(2) in the set), and so on. The propagation can be performed in the set of images 202 in a sequential order until the markers 310a and 310b are transferred to all images 202 in the set. The propagated measurement markers on the image 202_f(1) are shown as 312a and 312b.

In some instances, the propagation may skip one or more images 202 in the set. In general, the propagation can be performed to propagate measurement markers from an image 202, f(i), to a neighboring image 202 f(i+L) in the set, where L may be 1, 2, 3, or 4. While the propagation may be configured to skip one or more images 202 in the set by varying L, the two images 202_f(i) and 202 f(i+L) may become more dissimilar as L increases, and thus the registration may become less accurate. Therefore, it is preferred to perform registration between images 202 within close vicinity of each other.

Referring to FIG. 4, the scheme 400 can be implemented by the measurement marker propagation component 260. FIG. 4 provides a more detailed view of the registration process. The scheme 400 includes a registration and propagation component 410, which may include hardware and/or software configured to perform image registration and measurement marker propagation. In some aspects, the registration and propagation component 410 may register the image 202_f(0) and image 202_f(1) with respect to each other using model-based, mutual information-based, similarity-based registration techniques. Some examples similarity measurements may include sum of square difference (SSD) or sum of absolute difference (SAD) measurements. The registration and propagation component 410 may align image features of the image 202_f(1) to the image features of the image 202_f(0). While the image 202_f(1) and the image 202_f(0) may be parallel to each other, the image 202_f(1) and the image 202_f(0) may not be aligned to each other. Thus, the propagated marker 312a on the image 202_f(1) may not be at the same pixel location (e.g., a pixel (x, y) coordinate) as the marker 310a on the image 202_f(0). After the registration, the registration and propagation component 410 propagates the measurement markers 310a and 310b from the image 202_f(0) to the image 202_f(1).

In some aspects, to propagate the measurement markers 310a and 310b from the image 202_f(0) to the image 202_f(1), the registration and propagation component 410 may copy the measurement points (e.g., the locations of the measurement markers 310a and 310b) from the image 202_f(0) to the image 202_f(1). The registration and propagation component 410 may further adjust the copied marker locations to optimal locations. For instance, the initial placement of the measurement markers 310a and 310b on the image 202_f(0) may be based on locally maximizing or minimizing a certain cost function. In some instances, the cost function can be based on image brightness. Accordingly, the measurement point or marker locations copied to the image 202_f(1) may need to be re-evaluated at the vicinity of the copied measurement point or marker locations, for example, to optimize the cost function. The reevaluation or optimization of the copied measurement point or marker locations can be processed as a learning task to determine the most probable location of the measurement points on the image 202_f(1), which may be implemented via deep learning techniques as shown in FIG. 5.

For purpose of simplicity of illustration, FIG. 4 only illustrates the propagation of the marker 310a from the image 202_f(0) to the image 202_f(1) (shown as the marker 312a) in the dashed box. However, similar propagation may be shown for the marker 310b. As can be observed, the marker 310a is offset from the marker 312a on the image 202_f(1) if the image 202_f(0) is to be directly overlaid on top of the image 202_f(1) without registration. The output 402 shows the image 202_f(0) and the image 202_f(1) after registration, where the measurement marker 310a and 312a are spatially aligned and shown as 406a.

Referring to FIG. 5, the scheme 500 can be implemented by the measurement marker propagation component 260. The scheme 500 includes a deep learning network 510 trained to perform image registration and measurement marker propagation. As shown, the deep learning network 510 may be applied to the image 202_f(1) and the image 202_f(0) along with the measurement markers 310a and 310b. The deep learning network 510 generates an output 502 including the image 202_f(1) with the propagated markers propagated from the markers 310a and 310b on the image 202_f(0). For purpose of simplicity of illustration, FIG. 5 only illustrates the propagated marker 312a. However, similar propagation may be shown for the marker 310b.

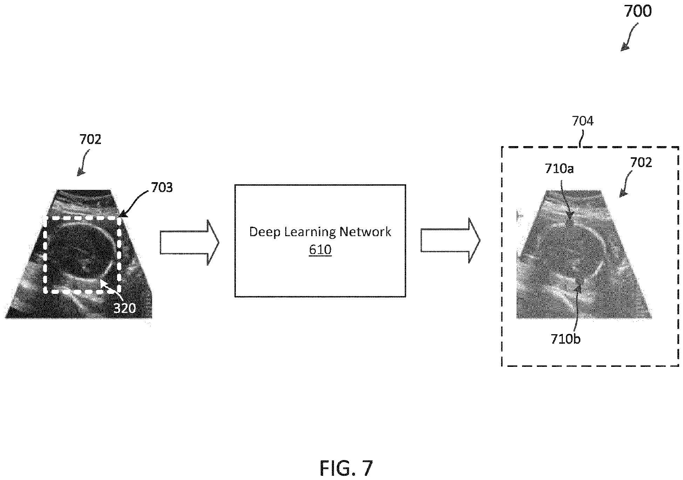

In some instances, the deep learning network 510 may include two CNNs, a first CNN for registration 512 and a second CNN for measurement marker propagation 514. The first CNN is trained to regress translational and rotational components of the registration process given a fixed image (e.g., the image 202_f(0)) and a moving image (e.g., the image 202_f(1)). The second CNN is trained to regress measurement point coordinates given an input ultrasound image. In some other instances, the deep learning network 510 may include the measurement CNN without the registration CNN. Registration may be performed between the images 202_f(0) and 202_f(1) prior to applying the deep learning network 510. In yet some other instances, the deep learning network 510 may include a single CNN trained to perform image registration and measurement marker propagation. The configuration and training of the deep learning network 510 are described in greater detail below in FIGS. 6 and 7, respectively.

Referring to FIG. 6, the configuration 600 can be implemented by the deep learning network 510. The configuration 600 includes a deep learning network 610 including one or more CNNs 612. For simplicity of illustration and discussion, FIG. 6 illustrates one CNN 612. However, the embodiments can be scaled to include any suitable number of CNNs 612 (e.g., about 2, 3 or more). The configuration 600 is described in the context of measurement marker propagation. However, the configuration 600 can be applied for measurement marker placement and/or image registration by training the deep learning network 610 for measurement marker placement and/or image registration as described in greater detail below.

The CNN 612 may include a set of $N$ convolutional layers 620 followed by a set of $K$ fully connected layers 630, where N and $K$ may be any positive integers. The convolutional layers 620 are shown as 620(i) to 620$_{(N)}$. The fully connected layers 630 are shown as 630(i) to 630$_{(K)}$. Each convolutional layer 620 may include a set of filters 622 configured to extract features from an input 602 including an image 202_f(L) and an image 202_f(i). The image 202_f(L) may include a view of an anatomical feature of interest along with measurement markers. For instance, the image 202_f(L) may correspond to the image 202_f(0) with measurement markers 310a and 310b placed on the fetal head 320 circumference by the user for a fetal head diameter measurement. The images 202_f(i) may correspond to images 202 in the set excluding the image 202_f(L). The values N and *K* and the size of the filters 622 may vary depending on the embodiments. In some instances, the convolutional layers 620(i) to $620_{(N)}$ and the fully connected layers 630(i) to $630_{(K-1)}$ may utilize a leaky rectified non-linear (ReLU) activation function and/or batch normalization. The fully connected layers 630 may be non-linear and may gradually shrink the high-dimensional output to a dimension of the prediction result 604.

The input images 202_f(L) and 202_f(i) may be passed through each layer 620, 630 in succession for feature extraction, analysis, and/or classification. Each layer 620, 630 may include weightings (e.g., filter coefficients for the filters 622 in the convolutional layers 620 and non-linear weightings for the fully-connected layers 630) that are applied to the input images 202_f(L) and 202_f(i) or an output of a previous layer 620 or 630. In some instances, the input images 202_f(L) and 202_f(i) may be input to the deep learning network 610 image-by-image. In some other instances, the images 202_f(L) and 202_f(i) may be input to the deep learning network 610 as a 3D volume data set.

The CNN 612 may output a prediction result 604 based on the input images 202_f(L) and 202_f(i). The prediction result 604 may include various types of data depending on the training of the deep learning network 610 as discussed in greater detail herein below. In some instances, the prediction result 604 may include the images 202_f(i), each with propagated measurement markers (e.g., the markers 312a and 312b) propagated from the measurement markers on the image 202_f(L). Additionally or alternatively, measurements may be made on the images 202_f(i) based on corresponding propagated markers and/or on the image 202_f(L) based on the user placed measurement markers and the prediction result 604 may include statistic measure of the measurements, such as a mean, a median, a variance, or a standard deviation. Additionally or alternatively, the prediction result 604 may include a confidence metric or confidence score computed based on a variance of the measurements. In general, the deep learning network 610 may be trained to output any suitable combination of the images 202 with the propagated measurement markers, the statistic metric, and/or the confidence metric in the prediction result 604.

Returning to FIG. 2, after propagating the measurement markers 310a and 310b onto the remaining images 202 (e.g., f(1) to f(N-1)) in the set of images 202, the measurement determination component 270 is configured to determine a final measurement 272 based on the output 262 (e.g., the images 202 with the propagated measurement marker) provided by the measurement marker propagation component 260.

Returning to FIG. 3, the measurement determination component 270 determines a measurement 272 for the diameter of the fetal head 320 based on the images 202 propagated with the measurement markers. FIG. 3 provides a side view of the multiple images 202 for the measurement determination. The images 202 are shown as dashed lines and the measurement markers are shown as solid circle on the circumference of the fetal head 320. The initially selected measurement markers 310a and 310b are shown for the image 202 f(0). The propagated measurement markers 312 and 312b are also shown for the image 202 f(1). The measurement determination component 270 may determine an optimal measurement plane for measuring a diameter or a maximum length of the fetal head 320 based on the measurement markers on all the images 202.

In some instances, the optimal measurement plane may be on an imaging plane of one of the images 202 (e.g., the image 202 f(1)). In some other instances, the optimal measurement plane may not be on any of the imaging planes used to acquire the images 202. For example, the optimal measurement plane may be between two of the acquired imaging planes (e.g., between imaging planes for the image 202 f(0) and the image 202 f(1)). Alternatively, the measurement determination component 270 may determine a cross-plane 340 for obtaining an optimal measurement for the diameter of the fetal head 320. The cross-plane 340 may intersect with one or more of the acquired imaging planes. As shown, the cross-plane 340 intersects the imaging plane of the image 202 f(2). The final measurement 272 may correspond to a distance between two points on the circumference of the fetal head 320 in the cross-plane 340.

In some aspects, the deep learning network 510 or 610 may be trained to create multi-planar reconstructions (MPRs) from the 3D volume (e.g., the 3D spatial data 242) and propagate the measurement markers from initial image 202 f(0) into all the MPRs as discussed in greater detail herein below. This enables the deep learning network 510 or 610 to make an optimal measurement (e.g., the final measurement 272) on a plane (e.g., the cross-plane 340) which is not an image plane of the images 202. Such measurement 272 would have been missed by the user since it is not on an imaging plane (indicated by the dotted lines). In some instances, the deep learning network 510 or 610 may include 2D convolutional layers (e.g., the convolutional layers 620) and may be applied to the 2D images 202 as shown in FIG. 6. In some other instances, the deep learning network 510 or 610 may include 3D convolutional layers (e.g., the convolutional layers 620) and may be applied to the 3D volume.

FIG. 7 is a schematic diagram of a deep learning network training scheme 700 for ultrasound image-based measurement, according to aspects of the present disclosure. The scheme 700 can be implemented by the system 100. To train the deep learning network 610 for ultrasound image-based measurement, a training data set (e.g., the image data set 140) can be created. The training data set may include image-measurement pairs. For each image-measurement pair, the training data set may associate an ultrasound image 702 of an anatomy (e.g., the fetal head 320) with a ground truth including measurement markers placed on the ultrasound image by an expert for a certain measurement (e.g., the diameter of the fetal head 320). The ultrasound images 702 may be images of a phantom, a live patient, and/or a cadaver acquire by a probe such as the probes 110 or 210. The deep learning network 610 can be applied to each image 702 in the data set, for example, using forward propagation, to obtain an output or a score for the input image. The coefficients of the filters 622 in the convolutional layers 620 and weightings in the fully connected layers 630 can be adjusted, for example, by using backward propagation to minimize a prediction error (e.g., a difference between the ground truth and the prediction result 704). The prediction result 704 may include predicted placement of measurement markers 710a and 710b on the image 702. In some instances, the coefficients of the filters 622 in the convolutional layers 620 and weightings in the fully connected layers 630 can be adjusted per input image-measurement pair. In some other instances, a batch-training process can be used to adjust the coefficients of the filters 622 in the convolutional layers 620 and weightings in the fully connected layers 630. For example, the prediction errors are accumulated for a subset of the image-measurement pairs are before the coefficients of the filters 622 in the convolutional layers 620 and weightings in the fully connected layers 630 are adjusted.

In some aspects, the training data set may cover a large population. For instance, for fetal imaging, the training dataset may include ultrasound images of fetus of different ages, different sizes, different weights, and/or rare abnormalities so that the deep learning network 610 can learn to predict fetal head 320 measurements for fetus of various conditions.

In some aspects, a region of interest (ROI) may be identified from the image 702 based on the measurement markers placed by the expert and the deep learning network 610 may be trained on image patches that include the ROI. For example, a portion 703 of the image 702 including the fetal head 320 (e.g., the ROI) is used as input to the deep learning network 610 for training.

In some aspects, the deep learning network 610 can provide a probability distribution map at the measurement marker locations and user measurements (e.g., the user selected marker location) are propagated as probability distributions. The probability distributions may be Gaussian distributions, where the peak of the probability distributions corresponds to the registered measurement points (e.g., the propagated measurement markers). The probability distribution of the predicted measurement markers can be reshaped based on the probability distribution of the user selected measurement marker locations. The peak of the reshaped probability distribution may provide a more accurate marker location. In this regard, the prediction may be formulated as a Bayesian inference problem as shown below:

$$p(x|y) = p(y|x)p(x), \hspace{3cm} (2)$$

where $p(x|y)$ represents the conditional probability distribution of a measurement marker location x given a new image y, $p(y|x)$ represents the conditional probability between the image y and the marker location x as predicted by the deep learning network 610 based on the observed image y, and $p(x)$ represents the prior distribution of x (i.e., user placed measurement points propagated onto the new image plane with a certain variance around them due to the registration accuracy and user uncertainty). If the probability distributions $p(y|x)$ and $p(x)$ are multiplied as shown in Equation (2), the peak location of the distribution $p(x|y)$ may provide an optimum location for a measurement marker on the image y. The probability functions are 2D functions, such as a Gaussian function, and thus the marker location may be determined based on the x, y location of the maxima of the final probability function (e.g., $p(y)$).

In some aspects, the deep learning network 610 may be trained using different initial conditions and/or different convergence conditions. The different training instances may produce different prediction results 704. The scheme 700 may compute a statistic measure (e.g., mean, media, variance, standard deviation) of the prediction results 704 from the different training instances. The prediction results 704 from the different training instances may have a Gaussian-like probability distribution. The scheme 700 may update the measurement marker location based on the peak of the probability distribution to provide an optimum marker location.

In some aspects, the deep learning network 610 may be trained to provide statistic metric and/or confidence metric for the user selected measurement marker locations and/or the predicted or propagated measurement marker. In this regard, the deep learning network 610 may be trained using a training data set (e.g., the training data set 140) that include image-metric pairs, each including an ultrasound image (e.g., the images 202) and a ground truth indicating measurement markers placed by experts and a corresponding statistic metric or confidence metric. The deep learning network 610 may be trained using similar per-frame update training or batch-training discussed above.

In some aspects, the deep learning network 610 may be trained to provide final measurement (e.g., the measurement 272 on the cross-plane 340 shown in FIG. 3). In this regard, the deep learning network 610 may be trained using a training data set (e.g., the training data set 140) that include image-measurement pairs, each including a set of ultrasound images (e.g., the images 202) in a 3D volume and a ground truth indicating an optimal measurement plane (e.g., the cross-plane 340) in the 3D volume as determined by experts and corresponding measurement (e.g., the measurement 272) on the measurement plane. The deep learning network 610 may be trained using similar per-frame update training or batch-training discussed above.

While the schemes 200-600 are described in the context of fetal imaging, similar mechanisms can be used in cardiac imaging for cardiac measurements. Some examples of cardiac measurements may include the length, the width, the size, and/or the volume of a heart chamber (e.g., a left ventricle, right ventricle, left atrium, right atrium, aorta, inferior vena cava). When the deep learning networks 510 or 610 is used for propagating measurement markers (e.g., the markers 310a, 310b, 312a, and/or 312b) for cardiac measurements, the deep learning networks 510 or 610 is trained using a training data set including image-measurement pairs, each including an ultrasound image of a cardiac structure and corresponding measurement. In general, each deep learning network may be trained for measurements of a specific type of anatomical structures (e.g., a fetal head circumference, a heart chamber diameter, femur length, abdominal circumference) since the deep learning network may predict salient measurement points using surrounding anatomical features extracted from the images and the training data set may cover a large population (e.g., different ages, weights, sizes, and/or medical conditions). The deep learning network may be trained to provide measurements of any suitable form, for example, including length, width, area, volume, size, radius, diameter, perimeter, and/or any suitable type of geometrical measurements.

Additionally, while the schemes 500-600 are described in the context of image-based learning and prediction for measurements of anatomical structures, where the inputs to the deep learning networks 510 and/or 610 are images, the deep learning networks 510 and/or 610 can be trained to operate on 3D spatial data set instead. Referring to the example shown in FIG. 6, the deep learning network 610 may receive an input 3D spatial data set including a data point in a 3D space for each pixel on each image 202 f(L) and f(i). Each data point, denoted as D, is defined by an x-y-z coordinate in the 3D space and an associated intensity value, which may be represented by D(x, y, z, intensity level). Additionally, the 3D spatial data set may include (x, y, z) coordinates of the initially selected measurement markers (e.g., the markers 310a and 310b) for the image 202_f(L).

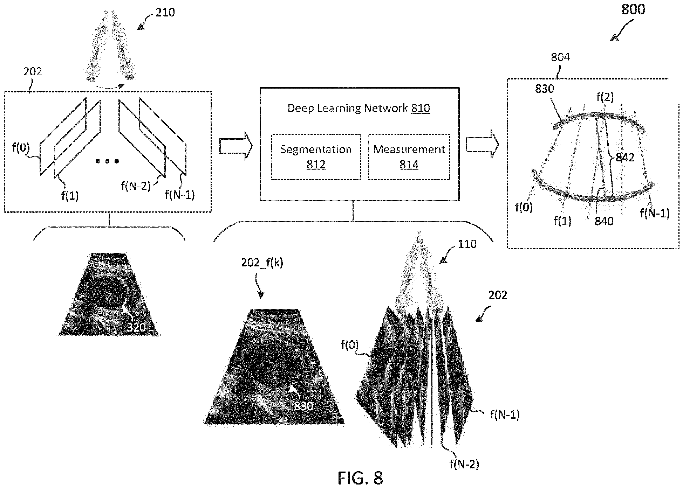

FIG. 8 is a schematic diagram of an automated deep-learning, ultrasound image-based measurement scheme 800, according to aspects of the present disclosure. The scheme 800 can be implemented by the system 100. The scheme 20 may utilize the scheme 800 for measurement marker placement, measurement marker propagation, and/or measurement determination in place of the measurement marker placement component 250, the measurement marker propagation component 260 and/or the measurement determination component 270.

The scheme 800 may apply a deep learning network 810 trained to perform image segmentation 812 and measurement 814. As shown, the deep learning network 810 may be applied to the set of images 202. The deep learning network 810 may be trained to segment a specific anatomical feature from a given input image and then determine a measurement for the segmented feature. In the example shown in FIG. 8, the input images 202 include a view of a fetal head 320. The deep learning network 810 is trained to segment the fetal head (shown as 830) from the images 202 and determine a diameter of the segmented fetal head segment 830. As shown, the deep learning network 810 predicted output 804 includes the segmented fetal head 830 on the images 202 (shown by the dashed lines) and a cross-plane 840 for measuring a diameter of the fetal head 830 (shown as a measurement 842). The cross-plane 840 may intersect the imaging plane of the image 202 f(2).

In some instances, the deep learning network 810 may include one CNN (e.g., the CNN 612) trained for the segmentation 812 and another CNN trained for the measurement 814. In some instances, the deep learning network 810 may include a single CNN trained for the segmentation 812 and the measurement 814. The deep learning network 810 may be trained using the scheme 700. The training data set may include image-segment pairs (e.g., an ultrasound image and a corresponding segmented feature) and/or image-measurement pairs (e.g., an ultrasound image and a corresponding measurement). Similarly, each deep learning network 810 may be trained for segmentation and measurement for a certain type of anatomical structures. While FIG. 8 illustrates the segmentation and the measurement using the deep learning network, in some other instances, the segmentation 812 can be performed using image processing and/or feature detection-based algorithms and the measurement 814 can be performed using a CNN.

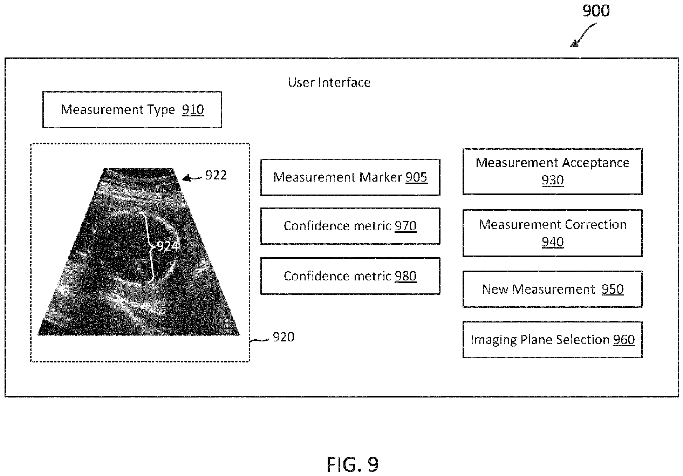

FIG. 9 is a schematic diagram of a user interface 900 for an automated ultrasound image-based measurement system, according to aspects of the present disclosure. The user interface 900 may be implemented by the system 100 and may be displayed on the display 132.

In some aspects, the user interface 900 may include a marker selection 905 and a measurement type selection 910. The marker selection 905 may allow a user to select an image (e.g., the images 202) and place measurement markers (e.g., the measurement markers 310a and 310b) on the selected image, for example, via a pop-up window. The measurement type selection 910 may be in the form of a drop-down menu or other user interface, where a user may select the type of measurements (e.g., a fetal head circumference, a fetal head maximum length measurement, a heart chamber length, width, size, and/or volume). The underlying system 100 may implement various deep learning networks (e.g., the deep learning networks 510, 610, and 810) that are trained for measurements of different anatomical structures, or different types of measurements for a particular type of anatomical structure, and/or segmentation of different types of anatomical structures. In some instances, the deep learning networks may also be trained to detect and segment the related anatomical structures in input images based on the measurement type selection 910.

In some aspects, the user interface 900 may include a measurement plane display panel 920, where the imaging plane 922 chosen by the deep learning network and the measurement 924 made by the deep learning network on the imaging plane 922 are displayed to the user. In some instances, the measurement plane display panel 920 may display one or more of the images with the propagated measurement markers and/or the initial image where the user places the measurement markers.

In some aspects, the user interface 900 may provide various options to the user regarding the measurements made by the deep learning network. In this regard, the user interface 900 includes a measurement acceptance selection 930, a measurement correction selection 940, a new measurement selection 950, and/or an imaging plane selection 960. The user may select the measurement acceptance selection 930 to accept the measurement 924 made by the deep learning network. The user may select the measurement correction selection 940 to correct the measurement 924 made by the deep learning network. The user may select the new measurement selection 950 to request the deep learning network to make another measurement. The user may select the image plane selection 960 to select a particular imaging plane for the measurement. In some aspects, the user's selections can be used to augment the training of the deep learning network.

In some aspects, the user interface 900 may display a confidence metric 970 and/or a statistic metric 980 determined by the deep learning network. As discussed above, the user may place measurement markers on an acquired image (e.g., the image 202 f(0)) and the deep learning network may propagate the measurement markers to neighboring images. The confidence metric 970 and/or the statistic metric 980 may provide a confidence measure or a statistic measure regarding the user selected measurement marker placement, respectively. The statistic metric 980 may include a mean, a median, a variance, a standard deviation of the user marker placement locations and the propagated marker locations. In some instances, the confidence metric 970 may be used to color code the display of the measurement 924 value. For example, the measurement 924 value may be displayed in red, yellow, or green to represent a low confidence, a medium confidence, or a high confidence, respectively.

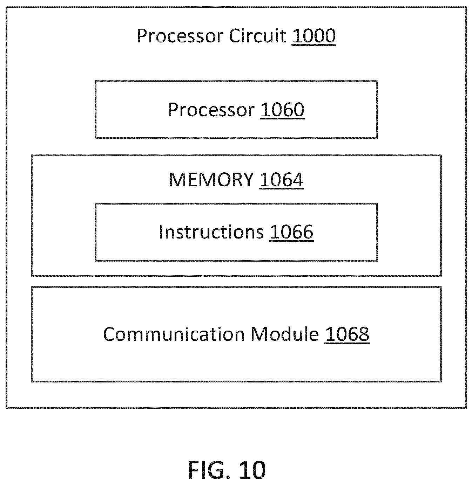

FIG. 10 is a schematic diagram of a processor circuit 1000, according to embodiments of the present disclosure. The processor circuit 1000 may be implemented in the probe 110 and/or the host 130 of FIG. 1. In an example, the processor circuit 1000 may be in communication with the transducer array 112 in the probe 110. As shown, the processor circuit 1000 may include a processor 1060, a memory 1064, and a communication module 1068. These elements may be in direct or indirect communication with each other, for example via one or more buses.

The processor 1060 may include a CPU, a GPU, a DSP, an application-specific integrated circuit (ASIC), a controller, an FPGA, another hardware device, a firmware device, or any combination thereof configured to perform the operations described herein, for example, aspects of FIGS. 2-10 and 12. The processor 1060 may also be implemented as a combination of computing devices, e.g., a combination of a DSP and a microprocessor, a plurality of microprocessors, one or more microprocessors in conjunction with a DSP core, or any other such configuration.

The memory 1064 may include a cache memory (e.g., a cache memory of the processor 1060), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1064 includes a non-transitory computer-readable medium. The memory 1064 may store instructions 1066. The instructions 1066 may include instructions that, when executed by the processor 1060, cause the processor 1060 to perform the operations described herein, for example, aspects of FIGS. 2-9 and 11 and with reference to the probe 110 and/or the host 130 (FIG. 1). Instructions 1066 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1068 can include any electronic circuitry and/or logic circuitry to facilitate direct or indirect communication of data between the processor circuit 1000, the probe 110, and/or the display 132. In that regard, the communication module 1068 can be an input/output (I/O) device. In some instances, the communication module 1068 facilitates direct or indirect communication between various elements of the processor circuit 1000 and/or the probe 110 (FIG. 1), the probe 210 (FIG. 2) and/or the host 130 (FIG. 1)

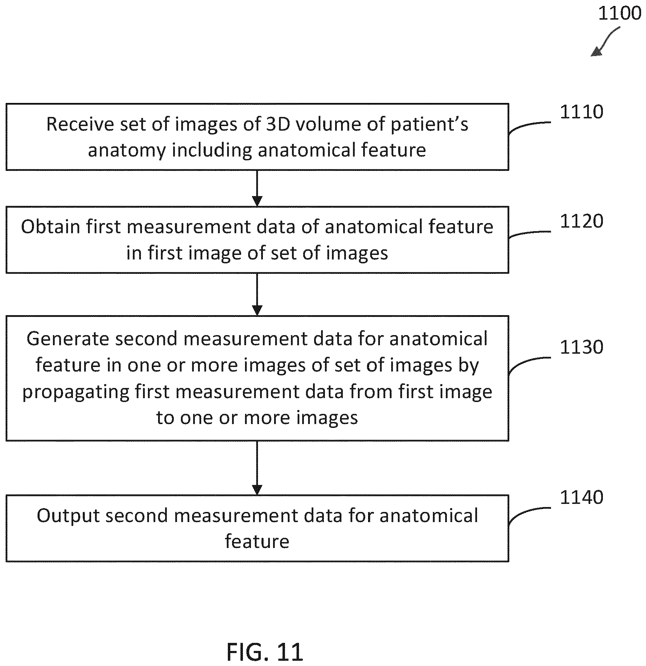

FIG. 11 is a flow diagram of a deep learning, ultrasound image-based measurement method 1100, according to aspects of the present disclosure. The method 1100 is implemented by the system 100, for example, by a processor circuit such as the processor circuit 1000, and/or other suitable component such as the probe 110 or 210, the processor circuit 116, the host 130, and/or the processor circuit 134. In some examples, the system 100 can include computer-readable medium having program code recorded thereon, the program code comprising code for causing the system 100 to execute the steps of the method 1100. The method 1100 may employ similar mechanisms as in the schemes 200, 300, 400, 500, 700, and/or 800 described above with respect to FIGS. 2, 3, 4, 5, 7, and/or 8, respectively, the configuration 600 described above with respect to FIG. 6, and the user interface 900 described above with respect to FIG. 9. As illustrated, the method 1100 includes a number of enumerated steps, but embodiments of the method 1100 may include additional steps before, after, and in between the enumerated steps. In some embodiments, one or more of the enumerated steps may be omitted or performed in a different order.

At step 1110, the method 1100 includes receiving, at a processor circuit (e.g., the processor circuits 134 and 1000) in communication with an ultrasound transducer array (e.g., the array 112), a set of images (e.g., the images 202) of a 3D volume of a patient's anatomy including an anatomical feature (e.g., a fetus, a heart chamber).

At step 1120, the method 1100 includes obtaining first measurement data of the anatomical feature in a first image of the set of images.

At step 1130, the method 1100 includes generating, at the processor circuit, second measurement data for the anatomical feature in one or more images of the set of images by propagating the first measurement data from the first image to the one or more images.

At step 1140, the method 1100 includes outputting, to a display (e.g., the display 132) in communication with the processor circuit, the second measurement data for the anatomical feature.

In some instances, the step 1120 includes receiving, from a user interface (e.g., the user interface 900) in communication with the processor circuit, the first measurement data including at least two measurement markers (e.g., the measurement markers 310a and 310b) across the anatomical feature.

In some instances, the set of images is associated with a plurality of imaging planes across the 3D volume of the patient's anatomy including the anatomical feature. The step 1130 includes determining 3D spatial data (e.g., the 3D spatial data set 242) for the first image and the one or more images based on positional data of the ultrasound transducer array with respect to the plurality of imaging planes and propagating the first measurement data from the first image to the one or more images based on the 3D spatial data.

In some instances, the method 1100 further includes receiving, from an inertial measurement tracker (e.g., the inertial measurement trackers 117 and 220) in communication with the processor circuit, inertial measurement data (e.g., positional information 222) associated with the ultrasound transducer array. The method 1100 further includes determining the positional data of the ultrasound transducer array with respect to the first image and the one or more images based on the inertial measurement data and an inertial-measurement-to-image transformation (e.g., transformation matrix $M_T$ in Equation (1)).

In some instances, the second measurement data includes propagated measurement markers (e.g., the measurement markers 312a and 312b) on the one or more images propagated from measurement markers placed on the first image.

In some instances, the method 1100 includes generating third measurement data for the anatomical feature based on the first measurement data and the second measurement data, wherein the third measurement data is associated with at least one of a first imaging plane of the plurality of imaging planes or a second imaging plane within the 3D volume different from the plurality of imaging planes. The method 1100 further includes outputting the third measurement data to the display. In some instances, the second imaging plane intersects the first imaging plane. For example, the first imaging plane (e.g., the measurement plane) is a cross-plane (e.g., the cross-planes 340 and 840). In some instances, the third measurement data includes at least one of the second measurement data, a distance between two measurement markers across the anatomical feature, a confidence metric of the first measurement data, a confidence metric of the second measurement data, a mean value of the first measurement data and the second measurement data, a variance of the first measurement data and the second measurement data, or a standard deviation of the first measurement data and the second measurement data.

In some instances, the method 1100 further includes providing a user with selection (e.g., the selections 930, 940, 950, and 960) associated with the third measurement (e.g., the measurements 272 and 924) via a user interface (e.g., the user interface 900).

In some instances, the step 1130 includes propagating the first measurement data from the first image to the one or more images based on image segmentation. In some instances, the step 1130 includes propagating the first measurement data from the first image to the one or more images using a predictive network (e.g., the deep learning networks 510, 610, and/or 810) trained for at least one of an image segmentation or a feature measurement.

In some instances, the predictive network is trained on a set of image-measurement pairs for the feature measurement, where each image-measurement pair of the set of image-measurement pair includes an image in a sequence of images of a 3D anatomical volume and a measurement of a feature of the 3D anatomical volume for the image. In some instances, the predictive network is trained on a set of image-segment pairs for the image segmentation, where each image-segment pair of the set of image-segment pair includes an image in a sequence of images of a 3D anatomical volume and a segment of a feature of the 3D anatomical volume for the image.

In some instances, the anatomical feature includes a fetal head, and the first measurement data and the second measurement data are associated with at least one of a circumference of the fetal head or a length of the fetal head.

In some instances, the anatomical feature includes a fetal head, and the first measurement data and the second measurement data are associated with at least one of a circumference of the fetal head or a length of the fetal head.

Aspects of the present disclosure can provide several benefits. For example, the use of a deep learning-based framework for automated anatomical feature measurement can provide a clinician with measurement assistance, reducing ultrasound examination time and/or user-dependency. Thus, the disclosed embodiments may provide more consistent, accurate measurements compared to conventional measurements that are dependent on the users. Additionally, the reconstruction of the 3D volume from the acquired images provide 3D information of the anatomical feature can allow for a more accurate measurement. Further, the use of a deep learning network trained to create MPRs and perform measurement based on the MPRs can further improve measurement accuracy, where measurements may not be limited to imaging planes acquired during acquisition.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An ultrasound imaging system comprising:
   a processor circuit (116) in communication with an ultrasound transducer array (112), the processor circuit configured to:

   receive, from the ultrasound transducer array, a set of images of a three-dimensional (3D) volume of a patient's anatomy including an anatomical feature, wherein the set of images is associated with a plurality of imaging planes across the 3D volume of the patient's anatomy including the anatomical feature;
   obtain first measurement data (252) of the anatomical feature in a first image of the set of images;
   generate second measurement data for the anatomical feature in one or more images of the set of images by propagating the first measurement data from the first image to the one or more images based on positional data of the ultrasound transducer array with respect to the plurality of imaging planes; and
   output, to a display in communication with the processor circuit, the second measurement data for the anatomical feature.

2. The system of claim 1, wherein the processor circuit configured to obtain the first measurement data is configured to:
   receive, from a user interface (900) in communication with the processor circuit, the first measurement data including at least two measurement markers across the anatomical feature on the first image.

3. The system of claim 1, wherein the processor circuit configured to generate the second measurement data is configured to:

   determine 3D spatial data for the first image and the one or more images based on the positional data of the ultrasound transducer array; and
   propagate the first measurement data from the first image to the one or more images based on the 3D spatial data.

4. The system of claim 3, further comprising:

   a probe including the ultrasound transducer array and an inertial measurement tracker, wherein the processor circuit is configured to:
   receive, from the inertial measurement tracker (220), inertial measurement data associated with the ultrasound transducer array and the plurality of imaging planes, and
   wherein the processor circuit configured to determine the 3D spatial data is configured to:
   determine the positional data of the ultrasound transducer array with respect to the plurality of imaging planes based on the inertial measurement data and an inertial-measurement-to-image transformation.

5. The system of claim 1, wherein the processor circuit is configured to:

   generate third measurement data for the anatomical feature based on the first measurement data and the second measurement data, wherein the third measurement data is associated with at least one of a first imaging plane of the plurality of imaging planes or a second imaging plane within the 3D volume different from the plurality of imaging planes; and
output, to the display, the third measurement data.

6. The system of claim 5, wherein the second imaging plane intersects the first imaging plane.

7. The system of claim 6, wherein the third measurement data includes at least one of the second measurement data, a distance between two measurement markers across the anatomical feature, a confidence metric of the first measurement data, a confidence metric of the second measurement data, a mean value of the first measurement data and the second measurement data, a variance of the first measurement data and the second measurement data, or a standard deviation of the first measurement data and the second measurement data,
optionally further comprising a user interface in communication with the processor circuit and configured to provide a selection associated with the third measurement data.

8. The system of claim 1, wherein the processor circuit configured to generate the second measurement data for the anatomical feature in the one or more images is configured to:
propagate the first measurement data from the first image to the one or more images based on image segmentation.

9. The system of claim 1, wherein the processor circuit configured to generate the second measurement data for the anatomical feature in the one or more images is configured to:
propagate the first measurement data from the first image to the one or more images using a predictive network trained for at least one of an image segmentation or a feature measurement.

10. The system of claim 9, wherein the predictive network is trained on a set of image-measurement pairs for the feature measurement, and wherein each image-measurement pair of the set of image-measurement pair includes an image in a sequence of images of a 3D anatomical volume and a measurement of a feature of the 3D anatomical volume for the image.

11. The system of claim 10, wherein the predictive network is trained on a set of image-segment pairs for the image segmentation, wherein each image-segment pair of the set of image-segment pair includes an image in a sequence of images of a 3D anatomical volume and a segment of a feature of the 3D anatomical volume for the image.

12. A method of ultrasound imaging, comprising:

receiving, at a processor circuit (116) in communication with an ultrasound transducer array (112), a set of images of a three-dimensional (3D) volume of a patient's anatomy including an anatomical feature, wherein the set of images is associated with a plurality of imaging planes across the 3D volume of the patient's anatomy including the anatomical feature;
obtaining, at the processor circuit, first measurement data (252) of the anatomical feature in a first image of the set of images;
generating, at the processor circuit, second measurement data for the anatomical feature in one or more images of the set of images by propagating the first measurement data from the first image to the one or more images based on positional data of the ultrasound transducer array with respect to the plurality of imaging planes; and
outputting, to a display in communication with the processor circuit, the second measurement data for the anatomical feature.

13. The method of claim 12, wherein the obtaining the first measurement data includes:
receiving, from a user interface (900) in communication with the processor circuit, the first measurement data including at least two measurement markers across the anatomical feature.

14. The method of claim 12, wherein the generating the second measurement data includes:

determining 3D spatial data for the first image and the one or more images based on positional data of the ultrasound transducer array with respect to the plurality of imaging planes; and
propagating the first measurement data from the first image to the one or more images based on the 3D spatial data using a predictive network trained for at least one of an image segmentation or a feature measurement.

**15.** The method of claim 14, further comprising:
receiving, from an inertial measurement tracker in communication with the processor circuit, inertial measurement data associated with the ultrasound transducer array, and determining the positional data of the ultrasound transducer array with respect to the first image and the one or more images based on the inertial measurement data and an inertial-measurement-to-image transformation.

**Patentansprüche**

**1.** Ultraschall-Bildgebungssystem, umfassend: eine Prozessorschaltung (116), die mit einem Ultraschallwandler-Array (112) kommuniziert, wobei die Prozessorschaltung dazu konfiguriert ist: empfangen von der Ultraschallwandleranordnung einen Satz von Bildern eines dreidimensionalen (3D) Volumens der Anatomie eines Patienten, einschließlich eines anatomischen Merkmals, wobei der Satz von Bildern mehreren Bildebenen über das 3D-Volumen des Patienten zugeordnet ist Anatomie einschließlich der anatomischen Merkmale; erhalten erster Messdaten (252) des anatomischen Merkmals in einem ersten Bild des Bildsatzes; zweite Messdaten für das anatomische Merkmal in einem oder mehreren Bildern des Satzes von Bildern erzeugen, indem die ersten Messdaten vom ersten Bild auf das eine oder die mehreren Bilder übertragen werden, basierend auf Positionsdaten des Ultraschallwandler-Arrays in Bezug auf die mehreren Bilder Flugzeuge; und ausgabe der zweiten Messdaten für das anatomische Merkmal an eine Anzeige, die mit der Prozessorschaltung in Verbindung steht.

**2.** System nach Anspruch 1, wobei die Prozessorschaltung, die zum Erhalten der ersten Messdaten konfiguriert ist, konfiguriert ist für: von einer Benutzerschnittstelle (900), die mit der Prozessorschaltung kommuniziert, die ersten Messdaten einschließlich mindestens zwei Messmarkierungen über das anatomische Merkmal auf dem ersten Bild zu empfangen.

**3.** System nach Anspruch 1, wobei die Prozessorschaltung, die zum Erzeugen der zweiten Messdaten konfiguriert ist, konfiguriert ist für: 3D-Raumdaten für das erste Bild und das eine oder die mehreren Bilder basierend auf den Positionsdaten des Ultraschallwandler-Arrays ermitteln; und weitergabe der ersten Messdaten vom ersten Bild an das eine oder die mehreren Bilder basierend auf den räumlichen 3D-Daten.

**4.** System nach Anspruch 3, weiterhin umfassend: eine Sonde, die das Ultraschallwandler-Array und einen Trägheitsmess-Tracker umfasst, wobei die Prozessorschaltung dazu konfiguriert ist: vom Trägheitsmess-Tracker (220) Trägheitsmessdaten empfangen, die mit dem Ultraschallwandler-Array und den mehreren Bildgebungsebenen verknüpft sind, und wobei die Prozessorschaltung, die zum Bestimmen der 3D-Raumdaten konfiguriert ist, konfiguriert ist für: bestimmen der Positionsdaten des Ultraschallwandler-Arrays in Bezug auf die mehreren Bildebenen basierend auf den Trägheitsmessdaten und einer Trägheitsmessung-zu-Bild-Transformation.

**5.** System nach Anspruch 1, wobei die Prozessorschaltung dazu konfiguriert ist: erzeugen dritter Messdaten für das anatomische Merkmal basierend auf den ersten Messdaten und den zweiten Messdaten, wobei die dritten Messdaten mit mindestens einer ersten Bildebene der mehreren Bildebenen oder einer zweiten Bildebene innerhalb des 3D verknüpft sind das Volumen unterscheidet sich von der Mehrzahl der Abbildungsebenen; und Ausgabe der dritten Messdaten auf dem Display.

**6.** System nach Anspruch 5, wobei die zweite Bildebene die erste Bildebene schneidet.

**7.** System nach Anspruch 6, wobei die dritten Messdaten mindestens eines der zweiten Messdaten, einen Abstand zwischen zwei Messmarkierungen über das anatomische Merkmal, eine Konfidenzmetrik der ersten Messdaten und eine Konfidenzmetrik der zweiten Messung umfassen Daten, ein Mittelwert der ersten Messdaten und der zweiten Messdaten, eine Varianz der ersten Messdaten und der zweiten Messdaten, oder eine Standardabweichung der ersten Messdaten und der zweiten Messdaten, optional weiterhin umfassend eine Benutzerschnittstelle, die mit der Prozessorschaltung kommuniziert und so konfiguriert ist, dass sie eine Auswahl im Zusammenhang mit den dritten Messdaten bereitstellt.

**8.** System nach Anspruch 1, wobei die Prozessorschaltung, die zum Erzeugen der zweiten Messdaten für das anatomische Merkmal in dem einen oder den mehreren Bildern konfiguriert ist, konfiguriert ist für: Weitergabe der ersten Messdaten vom ersten Bild an das eine oder die mehreren Bilder basierend auf der Bildsegmentierung.

**9.** System nach Anspruch 1, wobei die Prozessorschaltung, die zum Erzeugen der zweiten Messdaten für das anatomische Merkmal in dem einen oder den mehreren Bildern konfiguriert ist, konfiguriert ist für: weitergabe der ersten Messdaten vom ersten Bild an das eine oder die mehreren Bilder unter Verwendung eines Vorhersagenetzwerks, das für mindestens eine Bildsegmentierung oder eine Merkmalsmessung trainiert wurde.

10. System nach Anspruch 9, wobei das Vorhersagenetzwerk auf einen Satz von Bildmesspaaren für die Merkmalsmessung trainiert wird und wobei jedes Bildmesspaar des Satzes von Bildmesspaaren ein Bild in einer Bildfolge enthält eines anatomischen 3D-Volumens und eine Messung eines Merkmals des anatomischen 3D-Volumens für das Bild.

11. System nach Anspruch 10, wobei das Vorhersagenetzwerk auf einen Satz von Bildsegmentpaaren für die Bildsegmentierung trainiert wird, wobei jedes Bildsegmentpaar des Satzes von Bildsegmentpaaren ein Bild in einer Bildsequenz von enthält ein anatomisches 3D-Volumen und ein Segment eines Merkmals des anatomischen 3D-Volumens für das Bild.

12. Verfahren zur Ultraschallbildgebung, umfassend:

empfangen eines Satzes von Bildern eines dreidimensionalen (3D) Volumens der Anatomie eines Patienten, einschließlich eines anatomischen Merkmals, an einer Prozessorschaltung (116), die mit einem Ultraschallwandler-Array (112) in Verbindung steht, wobei der Satz von Bildern damit verknüpft ist eine Vielzahl von Bildebenen über das 3D-Volumen der Anatomie des Patienten einschließlich des anatomischen Merkmals; erhalten, an der Prozessorschaltung, erster Messdaten (252) des anatomischen Merkmals in einem ersten Bild des Satzes von Bildern; erzeugen, an der Prozessorschaltung, zweiter Messdaten für das anatomische Merkmal in einem oder mehreren Bildern des Bildsatzes durch Weitergabe der ersten Messdaten vom ersten Bild an das eine oder die mehreren Bilder basierend auf Positionsdaten des Ultraschallwandler-Arrays mit in Bezug auf die Vielzahl von Abbildungsebenen; und ausgeben der zweiten Messdaten für das anatomische Merkmal an eine Anzeige, die mit der Prozessorschaltung kommuniziert.

13. Verfahren nach Anspruch 12, wobei das Erhalten der ersten Messdaten umfasst: empfangen der ersten Messdaten, einschließlich mindestens zwei Messmarkierungen über das anatomische Merkmal, von einer Benutzerschnittstelle (900), die mit der Prozessorschaltung kommuniziert.

14. Verfahren nach Anspruch 12, wobei das Erzeugen der zweiten Messdaten umfasst: bestimmen von räumlichen 3D-Daten für das erste Bild und das eine oder die mehreren Bilder basierend auf Positionsdaten des Ultraschallwandler-Arrays in Bezug auf die mehreren Bildebenen; und weitergabe der ersten Messdaten vom ersten Bild an das eine oder die mehreren Bilder basierend auf den räumlichen 3D-Daten unter Verwendung eines Vorhersagenetzwerks, das für mindestens eine Bildsegmentierung oder eine Merkmalsmessung trainiert wurde.

15. Verfahren nach Anspruch 14, weiterhin umfassend: empfangen von Trägheitsmessdaten, die dem Ultraschallwandler-Array zugeordnet sind, von einem Trägheitsmess-Tracker, der mit der Prozessorschaltung in Verbindung steht, und Bestimmen der Positionsdaten des Ultraschallwandler-Arrays in Bezug auf das erste Bild und das eine oder die mehreren Bilder basierend auf der Trägheit Messdaten und eine Trägheitsmessung-zu-Bild-Transformation.

**Revendications**

1. Un système d'imagerie par ultrasons comprend: un circuit processeur (116) en communication avec un réseau de transducteurs à ultrasons (112), le circuit processeur étant configuré pour: recevoir, à partir du réseau de transducteurs à ultrasons, un ensemble d'images d'un volume tridimensionnel (3D) de l'anatomie d'un patient comprenant une caractéristique anatomique, l'ensemble d'images étant associé à une pluralité de plans d'imagerie à travers le volume 3D de l'anatomie du patient comprenant la caractéristique anatomique; obtenir les premières données de mesure (252) de la caractéristique anatomique dans une première image de l'ensemble d'images; générer des secondes données de mesure pour la caractéristique anatomique dans une ou plusieurs images de la série d'images en propageant les premières données de mesure de la première image à une ou plusieurs images sur la base des données de position du réseau de transducteurs à ultrasons par rapport à la pluralité de plans d'imagerie; et émettre, sur un écran en communication avec le circuit processeur, les secondes données de mesure pour la caractéristique anatomique.

**2.** Le système de la revendication 1, dans lequel le circuit processeur configuré pour obtenir les premières données de mesure est configuré pour: recevoir, à partir d'une interface utilisateur (900) en communication avec le circuit processeur, les premières données de mesure comprenant au moins deux marqueurs de mesure à travers la caractéristique anatomique sur la première image.

**3.** Le système de la revendication 1, dans lequel le circuit processeur configuré pour générer les secondes données de mesure est configuré pour: déterminer les données spatiales 3D pour la première image et l'une ou plusieurs images sur la base des données de position du réseau de transducteurs à ultrasons; et propager les premières données de mesure de la première image vers une ou plusieurs images sur la base des données spatiales 3D.

**4.** Le système de la revendication 3 comprend en outre: une sonde comprenant la matrice de transducteurs à ultrasons et un tracker de mesure inertielle, dans lequel le circuit processeur est configuré pour: recevoir, à partir du tracker de mesure inertielle (220), des données de mesure inertielle associées à la matrice de transducteurs à ultrasons et à la pluralité de plans d'imagerie, et dans lequel le circuit processeur configuré pour déterminer les données spatiales 3D est configuré pour: déterminer les données de position de la matrice de transducteurs à ultrasons par rapport à la pluralité de plans d'imagerie sur la base des données de mesure inertielle et d'une transformation mesure inertielle-image.

**5.** Le système de la revendication 1, dans lequel le circuit processeur est configuré pour: générer de troisièmes données de mesure pour la caractéristique anatomique sur la base des premières données de mesure et des deuxièmes données de mesure, les troisièmes données de mesure étant associées à au moins un premier plan d'imagerie de la pluralité de plans d'imagerie ou à un deuxième plan d'imagerie dans le volume 3D différent de la pluralité de plans d'imagerie; et transmettre les troisièmes données de mesure à l'afficheur.

**6.** Le système de la revendication 5, dans lequel le second plan d'imagerie coupe le premier plan d'imagerie.

**7.** Le système de la revendication 6, dans lequel les troisièmes données de mesure comprennent au moins l'une des deuxièmes données de mesure, une distance entre deux marqueurs de mesure à travers la caractéristique anatomique, une métrique de confiance des premières données de mesure, une métrique de confiance des deuxièmes données de mesure, une valeur moyenne des premières données de mesure et des deuxièmes données de mesure, une variance des premières données de mesure et des deuxièmes données de mesure, ou un écart type des premières données de mesure et des deuxièmes données de mesure, comprenant en outre une interface utilisateur en communication avec le circuit processeur et configurée pour fournir une sélection associée aux troisièmes données de mesure.

**8.** Le système de la revendication 1, dans lequel le circuit processeur configuré pour générer les deuxièmes données de mesure pour la caractéristique anatomique dans une ou plusieurs images est configuré pour: propager les premières données de mesure de la première image à une ou plusieurs images sur la base de la segmentation d'image.

**9.** Le système de la revendication 1, dans lequel le circuit processeur configuré pour générer les deuxièmes données de mesure pour la caractéristique anatomique dans une ou plusieurs images est configuré pour: propager les premières données de mesure de la première image à une ou plusieurs images en utilisant un réseau prédictif formé pour au moins l'une des segmentations d'image ou une mesure de caractéristique.

**10.** Le système de la revendication 9, dans lequel le réseau prédictif est entraîné sur un ensemble de paires de mesures d'images pour la mesure de la caractéristique, et dans lequel chaque paire image-mesure de l'ensemble de paires image-mesure comprend une image dans une séquence d'images d'un volume anatomique 3D et une mesure d'une caractéristique du volume anatomique 3D pour l'image.

**11.** Le système de la revendication 10, dans lequel le réseau prédictif est entraîné sur un ensemble de paires de segments d'image pour la segmentation d'image, dans lequel chaque paire image-segment de l'ensemble de paires de segments d'image comprend une image dans une séquence d'images d'un volume anatomique 3D et un segment d'une caractéristique du volume anatomique 3D pour l'image.

**12.** Une méthode d'imagerie par ultrasons comprenant: la réception, au niveau d'un circuit processeur (116) en communication avec un réseau de transducteurs à ultrasons (112), un ensemble d'images d'un volume tridimensionnel (3D) de l'anatomie d'un patient comprenant une caractéristique anatomique, dans lequel l'ensemble d'images est associé à une pluralité de plans d'imagerie à travers le volume 3D de l'anatomie du patient comprenant la caractéristique anatomique; obtenir, au niveau du circuit processeur, les premières données de mesure (252) de la caractéristique anatomique dans une première image de l'ensemble d'images; générer, au niveau du circuit processeur, les secondes données de mesure pour la caractéristique anatomique dans une ou plusieurs images de l'ensemble d'images en propageant les premières données de mesure à partir de la première image à une ou plusieurs images sur la base des données de position du réseau de transducteurs à ultrasons par rapport à la pluralité de plans d'imagerie; et l'émission, sur un écran en communication avec le circuit de processeur, des deuxièmes données de mesure pour la caractéristique anatomique.

13. La méthode de la revendication 12, dans laquelle l'obtention des premières données de mesure comprend: la réception, à partir d'une interface utilisateur (900) en communication avec le circuit processeur, les premières données de mesure comprenant au moins deux marqueurs de mesure en travers de la caractéristique anatomique.

14. La méthode de la revendication 12, dans laquelle la production des secondes données de mesure comprend: la détermination des données spatiales 3D pour la première image et une ou plusieurs images sur la base des données de position du réseau de transducteurs à ultrasons par rapport à la pluralité de plans d'imagerie; et la propagation des premières données de mesure de la première image à une ou plusieurs images sur la base des données spatiales 3D à l'aide d'un réseau prédictif entraîné pour au moins l'un des deux types de segmentation d'image ou de mesure de caractéristiques.

15. La méthode de la revendication 14 comprend en outre: la réception, à partir d'un tracker de mesure inertielle en communication avec le circuit processeur, de données de mesure inertielle associées à la matrice de transducteurs à ultrasons, et la détermination des données de position de la matrice de transducteurs à ultrasons par rapport à la première image et à une ou plusieurs images sur la base des données de mesure inertielle et d'une transformation mesure inertielle-image.

100

Probe 110

Inertial Measurement
Tracker
117

105

Transducer
Array
112

Beamformer
114

Processor
116

Communication
Interface
118

Host 130

Display
132

Processor
134

Communication
Interface
136

120

Memory 138

Image Data Set
140

FIG. 1

FIG. 2

FIG. 3

FIG. 4

202_f(0)

202_f(1)

500

310a

320

320

502

Deep Learning Network 510

Registration 512

Measurement
Marker Propagation
514

510a

320

FIG. 5

FIG. 6

FIG. 7

FIG. 8

900

User Interface

Measurement Type  910

922

924

920

Measurement Marker 905

Confidence metric 970

Confidence metric 980

Measurement Acceptance
930

Measurement Correction
940

New Measurement  950

Imaging Plane Selection 960

FIG. 9

Processor Circuit <u>1000</u>

Processor <u>1060</u>

MEMORY <u>1064</u>

Instructions <u>1066</u>

Communication Module <u>1068</u>

FIG. 10

1100

Receive set of images of 3D volume of patient's anatomy including anatomical feature — 1110

Obtain first measurement data of anatomical feature in first image of set of images — 1120

Generate second measurement data for anatomical feature in one or more images of set of images by propagating first measurement data from first image to one or more images — 1130

Output second measurement data for anatomical feature — 1140

FIG. 11

**EP 4 061 231 B1**

**Patent documents cited in the description**

- US 2017325783 A1
- US 2008221446 A1